Europäisches Patentamt

(19) European Patent Office

Office européen des brevets

(11) Publication number: **0 245 552 B1**

## (12) EUROPEAN PATENT SPECIFICATION

(45) Date of publication of patent specification:
09.01.91 Bulletin 91/02

(51) Int. Cl.⁵: **A61B 17/22, A61B 17/36**

(21) Application number: **86303746.1**

(22) Date of filing: **16.05.86**

54 Laser catheter device.

43 Date of publication of application:
19.11.87 Bulletin 87/47

45 Publication of the grant of the patent:
09.01.91 Bulletin 91/02

84 Designated Contracting States:
AT BE CH DE FR GB IT LI LU NL SE

56 References cited:
EP-A- 0 069 930
EP-A- 0 194 856
WO-A-84/04879
DE-A- 2 821 376
US-A- 3 096 767
US-A- 3 291 749
US-A- 4 316 467
US-A- 4 336 809
IEEE TRANSACTIONS ON BIOMEDICAL
ENGINEERING, vol. BME-33, no. 2, February
1986, pages 117-132, IEEE, New York, US; J.L.
GEHRICH et al.: "Optical fluorescence and its
application to an intravascular blood gas
monitoring system"

73 Proprietor: GV Medical, Inc.
3750 Annapolis Lane
Minneapolis Minnesota 55441 (US)

72 Inventor: Kosa, Nadhir B.
3750 Annapolis Lane
Minneapolis Minnesota 55441 (US)
Inventor: Burke, James J.
4850 Via Serenidad
Tucson Arizona 85718 (US)
Inventor: Moore, Gary L.
3750 Annapolis Lane
Minneapolis Minnesota 55441 (US)

74 Representative: MacGregor, Gordon et al
ERIC POTTER & CLARKSON St. Mary's Court
St. Mary's Gate
Nottingham, NG1 1LE (GB)

## Description

The present invention relates to a laser catheter device and especially to a laser enhanced transluminal angioplasty catheter system.

A laser enhanced transluminal angioplasty catheter allows obstructions in blood vessels to be treated with exposure to laser energy to obtain either a partial reduction and/or elimination of the obstruction by means of such exposure. An optical fiber is utilized for transmitting the laser beam energy from the generator onto the obstruction located in the zone to be treated. The optical fiber can also be utilized for the purpose of providing illumination and optical viewing. Laser enhanced transluminal angioplasty catheter devices are considered valuable tools for the treatment of commonly encountered forms of arteriosclerosis and the like.

Atheroclerosis is among the more commonly encountered forms of arteriosclerosis as it relates to the human heart and circulatory system, which typically has been treated by drugs, angioplasty catheterization, and also through open heart bypass surgical procedures. Of these various forms of treatment, angioplasty catheterization has been found to be a treatment of choice in certain situations. Such treatment normally involves initially bringing a balloon-tipped catheter proximate the material forming the obstruction matter in the vessel, with the distal tip catheter normally being forced through the obstruction and the balloon portion is thereafter inflated so as to cause dilation of the obstruction. This procedure is effective in reopening the blood vessel and restoring substantially normal circulation in many cases. This procedure is, however, especially dependent upon the skill of the cardiologist, and particularly as that skill pertains to manipulation and ultimate direction and control of the catheter. Normally, some assistance is provided through fluoroscopy techniques, typically through the incorporation of radiopaque members along the catheters. It is further recognized that angioplasty catheterization is frequently a procedure limited to those patients having obstructions which have not totally occluded the blood vessels to the point where the nominal diameter of the distal tip of the catheter would otherwise prevent passage of the catheter through the obstruction prior to dilation of the vessel.

Because of its application to surgical procedures, laser enhanced transluminal angioplasty catheters have been developed. In such devices, the catheter is provided with a source of laser energy, and this laser energy is directed to the site along optical fibers.

By way of background, dilating catheters are known, having been disclosed in U.S. Patent Nos. 4,040,413 ; 4,271,839 ; and 4,299,226. Catheter devices including optical fibers are disclosed in U.S. Patent Nos. 3,123,066 ; 3,136,310 ; 3,858,577 ; 4,146,019 ; and 4,273,019. Also, laser enhanced catheters are disclosed in U.S. Patent Nos. 3,467,098 ; 3,538,919 ; 3,843,865 ; and 4,266,548.

An additional example of multi-technology catheters is described in U.S. Patent 4,207,874 and the American Journal of Cardiology, 50 : 80, 81 (December 1982). In U.S. Patent 4,207,874, and of which the American Journal of Cardiology article is an elaboration, a fiber optic directable catheter is disclosed, with the device including a bundle of laser transmitting fibers and a centrally disposed lumen for permitting the suction removal of vaporized waste material after exposure to laser beam energy. Upon completion of the tunneling procedure, blood samples which are collected in a transparent external collection reservoir visually indicate the extent of completion of the procedure to the operator.

In laser enhanced transluminal angioplasty catheters, it is desirable for the operator to determine and to be reasonably confident that the laser energy being directed to the site is within certain predetermined desirable limits, that is, the energy is known to exceed a certain base or minimum threshold level, while not exceeding a certain upper limit. An indication that the laser beam energy is within predetermined limits will enable the procedure to be more reliable, expedient, reproducible, and efficacious. Inasmuch as optical fibers are being employed, along with other electro-mechanical and optical systems, proper evaluation of the operating parameters is desired. For example, the rupture, fracture, or occurrence of damage to the optical fiber may reduce the energy level available at the distal tip of the catheter to such an extent that the procedure would be generally ineffective. On the other hand, excessive quantities of laser beam energy available at the distal tip of the catheter may adversely affect the quality of the procedure.

The preferred embodiment of the present invention utilizes a control system which is interposed along the optical fiber segments which are provided to transmit laser energy from the generator to the distal tip. The control system includes an electromechanical shutter and a beam splitter disposed along the beam path. An attenuator and a focusing lens are located orthogonal to the energy beam, and a predetermined proportion of the energy is reflected and/or sampled by the beam splitter and directed into the attenuator and focusing lens and onto a first detector. The detector is, in turn, coupled to a power level monitor. On the catheter side of the beam splitter, and also orthogonal to the beam is an interference filter and a second focusing lens. At the distal tip of the optical fiber there is disposed a fluorescent element. The term "fluorescent" is intended to be used in a comprehensive sense, such as the response generated in such an element when irradiated with electromagnetic radiation of a given wavelength, with the response being the emission of radiation at a second and longer wavelength. One particularly desirable optically reactive device is a sapphire doped to fluoresce when transmitting laser energy. Since the fluorescent energy which is generated by the incident laser energy is at a wavelength significantly different from that of the laser energy, the fluorescent energy is transmitted or reflected back through the optical fiber and onto the catheter facing surface of the beam splitter. The fluorescent energy is then reflected by the catheter facing surface of the beam splitter (dichroic filter) into a focusing lens and thence onto a second detector. The output of the second detector is typically amplified and fed into a threshold detector. The output of the threshold detector is, in turn, coupled to the shutter system as well as to the power supply of the laser generator.

In the event the distal portion and/or distal tip of the optical fiber becomes damaged, the quantity or output of fluorescent energy generated by the optically reactive means will become diminished and the threshold detector will respond to the drop with a reduction in signal amplitude. This change, as detected, will create a signal which may be fed through a feedback loop which will, in turn, either shut off the laser generator and/or close the shutter system. The system of the present invention permits simultaneous monitoring of the output of the laser generator and the output of the optical fiber. In certain applications, an attenuator may be placed in the laser beam path in front of the shutter system. The ratio of the optical fiber output relative to the laser generator output is monitored continuously, and in the event the ratio falls outside of a predetermined range because of a failure of a system component, the entire system may be automatically shut down.

The present invention allows the feedback of information from the distal tip of the catheter without the need for additional optical fibers or wires to be employed in the catheter. Furthermore, the fiber optic tip at the end of the catheter is properly protected. The present invention further allows simultaneous measuring and/or comparing of the laser generator output and the catheter output, with the response time of the feedback system being extremely fast for proper system monitoring. In the event of a malfunction, or if the output deviates from a pre-established or predetermined range, the system may be immediately shut down.

In addition to the control function, the placement of a fluorescing lens at the distal tip of the optical fiber provides a convenient means for focusing and/or diverging the laser beam energy. This lens also provides protection for the tip of the optical fiber. Such a lens also makes it possible to monitor the temperature level existing at the distal tip of the optical fiber. Temperature monitoring is an available feature which may be accomplished by intermittent pulsing of the laser along with a measurement of the response time of the incident fluorescent energy. The output of the catheter may be monitored relative to the laser output so as to assure operation at the appropriate and desired level.

EP-A-0069930 discloses in Fig.3 a laser system for a medical application incorporating an optical fiber in which detectors are located at positions remote from the laser energy source and from the laser output position. The detectors monitor an abnormal intensity of laser energy in the fiber e.g. due to optical fiber damage. The pre-characterising clause of Claim 1 is based on this disclosure.

The characterising features of the invention are as set out in the characterising clause of Claim 1.

WO-A-8404879 discloses a medical appliance with a head heated by laser energy. Means is provided to detect the heat energy generated by the head. The laser beam passes down an optical fiber and means is provided at the proximal end of the fiber to intercept heat energy, received down the fiber from the head, and direct this energy to a pyrometer so as to measure the temperature of the head. In order to measure the temperature, the laser is deactivated and the intercepting means is moved from a first position, for passing the laser beam, to a second position for directing the heat energy to the pyrometer.

Reference is now made to the accompanying drawings, wherein :

Figure 1 is a schematic diagram illustrating one embodiment of the present invention, and illustrating simultaneously monitors or measures the output energy from the laser generator as well as the energy received through the transmission system and accordingly available at the distal tip of the catheter ;

Figure 2 is a schematic diagram illustrating the details of the optical unit utilized in the laser enhanced transluminal angioplasty catheter feedback system with the energy feedback being available from the distal tip of the catheter ; and

Figure 3 is a fragmentary detail schematic diagram of the distal tip portion only of the catheter assembly.

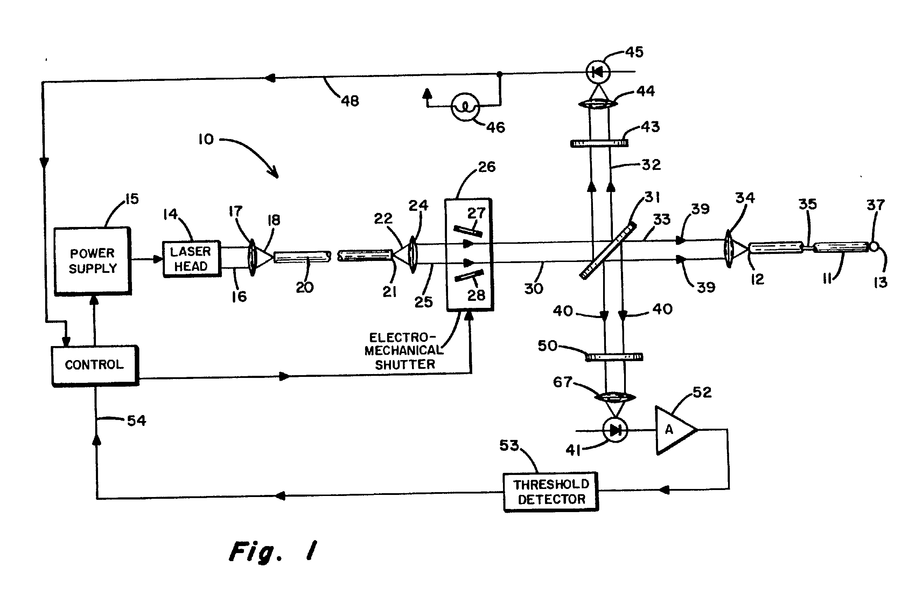

The laser enhanced transluminal angioplasty catheter system generally designated 10 (Figure 1) includes a transluminal angioplasty catheter member 11 having a proximal end 12 and a distal tip end 13, and with the catheter incorporating optical fibers for transmission of laser beam energy therethrough. Laser energy is provided from laser head 14, driven by power supply and control 15. Laser heads for the generation of laser energy for laser enhanced transluminal angioplasty catheter devices are commercially available, as are power supply and control systems therefor. The energy generated at the laser head 14 is transmitted through the wall of the head, along column 16, and into focusing lens 17. Focusing lens 17 directs the energy along the column shown as at 18, and into a first optical fiber segment as at 20. Optical fiber 20 is of a convenient length, such as a length of, for example, 10 meters. The terminal end 21 of optical fiber segment 20 is designed to pass laser energy along an energy cone as at 22, and into focusing lens 24. The beam passing through lens 24 is preferably arranged in a collimated form or parallel path as at 25, and passed into electromechanical dual shutter 26. In normal operation, dual shutter 26 permits the beam to pass therethrough, as is indicated in Figure 1, and in the event of any malfunction as will be described hereinafter, electromechanical dual shutter 26 will close its panel members or vanes 27 and 28 so as to interrupt the passage of laser beam energy therethrough. The laser energy exiting shutter 26 is shown in beam form 30, and passes onto beam splitter 31. Beam splitter 31 may conveniently be and is preferably a dichroic filter arranged for substantial or maximum transmission of incident laser beam energy. The small portion or sample of incident laser energy which is reflected off the surface of beam splitter 31 is indicated as at 32, with the balance of the energy passing through splitter 31 and along beam column shown at 33. Beam column 33 passes through focusing lens 34, and into a second segment of optical fibers 35 contained within a lumen formed in laser enhanced transluminal angioplasty catheter 11. At the distal tip of the catheter 11 is a fluorescent element for generating radiant energy at a wavelength significantly different from that of the incident laser beam energy. Typically, the fluorescent means may be a sapphire element containing a dopant which generates fluorescent energy or other radiant energy which is emitted at a wavelength significantly different from that of the incident laser beam energy. The sapphire element, such as optically reactive or doped sapphire element 37 is, of course, commercially available. One such useful dopant is chromium. While a sapphire element containing a dopant has been found to be desirable for use as the material of construction for element 37, it will, of course, be appreciated that other fluorescent substances may be employed as well. The material must be highly transmissive to energy within the wavelength of the laser beam, and further it must have the property of generating fluorescent energy or other detectable radiant energy at a wavelength significantly different from that of the incident laser beam energy.

The radiant energy generated by the fluoresent sapphire element 37 is received by and passed along optical fiber 35, in the direction indicated by arrows 39-39, and onto the catheter facing surface of beam splitter 31. The beam splitter (dichroic filter) which in this case, reflects the energy of the wavelength generated by the fluorescent member, passes along the path indicated by arrows 40-40 and ultimately received at the active surface of detector 41. This portion of the apparatus will be described in detail hereinbelow.

Turning now to the means for monitoring incident laser light from the laser generator or head 14, this sample of the overall output energy of the laser passes along beam column 32 through attenuator 43, focusing lens 44, and onto the active surface of radiant energy detector 45. A power level indicator as at 46 is in circuit with detector 45, thus providing an indication of the power level of incident laser energy received at the incoming surface of beam splitter 31. Furthermore, the output of detector 45 is coupled along line 48 to power supply and power control 15 through the control logic. Detectors such as detector 45, power level indicator 46, and the corresponding input to power supply and control 15 are commercially available.

Turning now to the reflected light which is generated by optically reactive member 37, received upon and transmitted from the second surface of beam splitter 31, such as is shown at 40-40, an interference filter is shown in the path as at 50, with this filter being adapted for maximun transmission of incident radiation at about 6940 Angstroms. This filter will typically have its peak transmission at the range of maximum generation of the optically reactive member 37. The signal generated by detector 41 is passed through a typical pre-amplifier as at 52, for transmission to threshold detector 53. The output of threshold detector 53 is normalized with respect to the output laser energy detector and is transmitted to separate logic controls, one being an input to electromechanical dual shutter 26, the other being an input, as at 54, to power supply and control 15. As has been previously indicated, if the normalized output of threshold detector 53 is outside of prescribed and desired limits, the active vanes 27 and 28 of shutter 26 are closed so as to totally interrupt and shut down the transmission of laser beam energy into the catheter 11.

It will be appreciated, therefore, that the laser beam energy passing between focusing lens 24 and focusing lens 34 is, in effect, passing through an evaluation zone for controlling the amount of energy available in the overall system, and also available at the distal tip of the laser enhancing transluminal angioplasty catheter 11.

Attention is now directed to Figure 2 of the drawings wherein the optical unit for the laser catheter is shown in greater detail. Similar numerals are utilized to identify similar items from that shown in Figure 1 where practical.

In the system of Figure 2, and within the laser unit block is laser head 15A driven by power supply 15B so as to generate a column of laser beam energy as at 16. Focusing lens 17 provides a means for focusing and directing the beam into the first optical fiber segment as at 20, with segment 20 being typically 10 meters in length.

The control unit block commences generally at the terminal end of the first optical fiber segment, as also shown in Figure 1. Lens 24 is employed to collimate the beam and pass the beam through an attenuator 60. Attenuator 60 is utilized to control the amount of the transmitted power through the system. Light passing through attenuator 60, as previously indicated, passes through electromechanical dual shutter 26 and onto the first surface of beam splitter 31. In this embodiment, beam splitter 31 is a dichroic filter, with such dichroic filters being, of course, commercially available. Dichroic filter 31 is selected so as to provide for maximum transmission of laser light, with only a small percentage being reflected along path 32, as previously set forth in the embodiment of Figure 1. Attenuator 43 is employed to control the amount of energy incident to and received upon detector 45. The output from detector 45, as indicated previously, is conveniently displayed on power level indicator 46, and ultimately received at the first control input 15C to power supply 15B.

The laser enhanced transluminal angioplasty catheter 11 as illustrated in Figure 2 is provided with one or more additonal lumens for the transmission of fluids, with the lead-in to the fiuid transmitting lumen being shown at 61. A protective shield or tubing for catheter 11 is illustrated at 62, with the tubing for the laser enhanced transluminal angioplasty catheter device 11 being preferably formed of an FDA approved material or substance such as, for example, polyethylene or equivalent. A metallic or glass sleeve is provided at 63 for receiving optical fibers and the sapphire element. In the application of a glass tube, additional radiopaque bands, such as at 64 and 65, are employed in order to assist in determining and viewing the location of the distal tip portion of laser enhanced transluminal angioplasty catheter 11, and also, in certain instances, may be utilized to locate the position of the distal tip of the optical fiber portions in those systems wherein the optical fibers may extend beyond the distal tip of the catheter sleeve portion, such as beyond polyethylene sleeve portion 11A.

In order to protect the surface of fiber or fibers 35, a buffer sheath may be utilized to encapsulate or coat the outer surface. Typically, this buffer layer may be one or more films or layers of a polyamide material, and may, in certain instances, contain a UV marker additive. Also, in certain applications, it may be desirable to provide an inner buffer film or layer for the polyethylene sleeve portion 11A. Such material may be formed of a compatible polymeric material such as an epoxy material or the like. It may be desirable, in certain instances, to provide this inner film or layer with a UV marker.

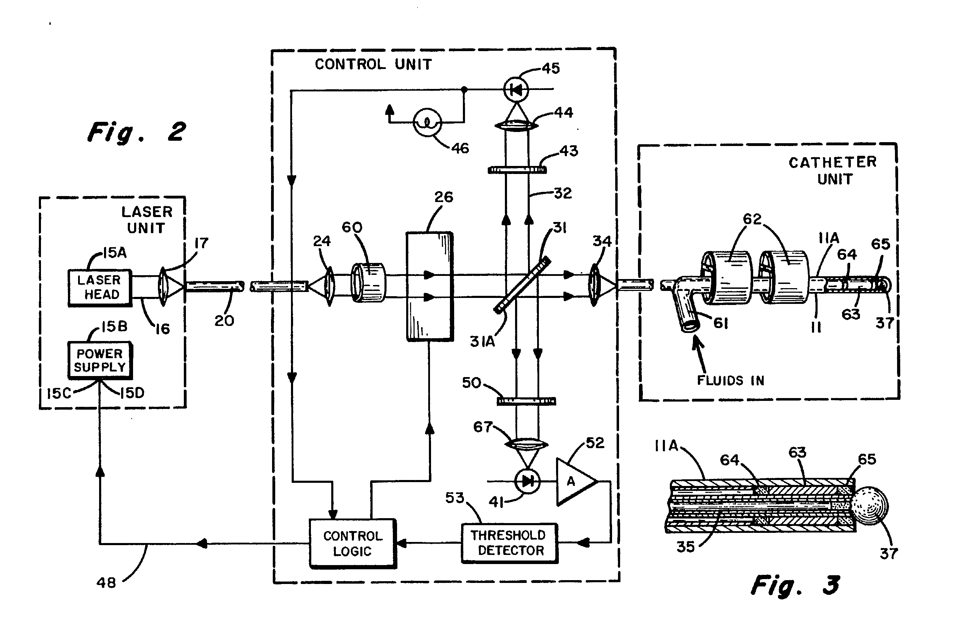

With attention being directed to Figure 3, and because of limitations of draftsmanship, the individual multi-layer arrangements are illustrated in fragmentary cut-away form.

As illustrated in Figure 1, reflected light from optically reactive and responsive element 37 is reflected from surface 31A of beam splitter (dichroic filter) 31 and through interference filter 50 and through focusing lens 67 and into detector element 41. The output of threshold detector 53, as previously indicated in connection with Figure 1, provides inputs to electromechanical shutter 26 as well as to power supply 15B, such as at the second control input 15D to power supply 15B.

As an alternative layout for the system, beam energy from the laser unit may be passed directly onto a beam splitter, with first and second detectors being employed to deliver signals to the control monitor, such as an electromechanical shutter system. On the catheter side of the beam splitter, a focusing lens is provided to carry the laser beam energy into the input of a first optical fiber segment, which terminates through connectors into the control monitor. The output from the control monitor is, in turn, delivered into the second optical fiber segment, and thence into the distal tip end of the catheter unit. Such an arrangement provides an advantage of employing one less alignment mechanism, and eliminates certain lenses.

## Claims

1. A laser medical device (10) including a transmitting column (16) between a laser source (14) and an appliance (11), the device having a control system provided with means for continuously monitoring the intensity of the laser beam at points positioned along the device, the control system having first and second detectors (45,41) positioned along the transmitting column, and control means (26) actuable is response to the first or second detector to interrupt the passage of the laser beam to the appliance upon the occurrence of an anomolous level of the laser beam intensity at the respective detector (45) CHARACTERISED in that said appliance is a catheter of the type for insertion in a blood vessel for transmitting the laser beam onto an obstruction, the control system monitors the intensity of the laser beam at the distal tip (13) of the catheter, the control system includes a beam splitter (31) for diverting a portion of the laser beam directed to the distal tip (13) on to the first dectector (45), fluorescent optically reactive means (37) disposed at the distal tip in the path of said laser beam for transmission of substantially all of the laser beam through the reactive means, said reactive means serving to generate radiant energy of a wavelength significantly different from that of the laser beam from a portion of said laser beam and being arranged such that said generated radiant energy is passed through the transmitting column to the beam splitter (31) during transmission of the laser beam, the second detector (41) being arranged to receive the radiant energy diverted by the beam splitter, and the control system serves to interrupt the passage of the laser beam to the catheter upon the ratio of the transmitting column output to the laser source output falling outside a predetermined range.

2. A laser catheter device according to Claim 1, wherein the control means (26) comprises shutter means for interrupting the passage of the laser beam between the source (14) and the beam splitter (31).

3. A laser catheter device according to Claim 1 or 2, which is a laser-enhanced transluminal angioplasty catheter, the transmitting column including an optical fiber (35) for transmission of the laser beam from the proximal end to the distal tip end of the catheter, the beam splitter (31) being located at the proximal end of said catheter.

4. A laser catheter device according to Claim 1, 2 or 3 wherein the beam splitter (31) is a dichroic filter.

5. A laser catheter device according to Claim 4 wherein the fluorescent element (37) contains a dopant and is arranged to fluoresce at a wavelength greater than that of the incident laser beam.

6. A laser catheter device according to any preceding claim wherein the transmitting column (16) includes spaced apart fiber optical segments (20,35) and the beam splitter (31) is interposed between said segments.

## Ansprüche

1. Medizinische Laservorrichtung (10) mit einem Durchlaß oder Übertragungsbündel (16) zwischen einer Laser (strahl)-quelle (14) und einem Gerät (11), wobei die Vorrichtung ein Steuersystem mit Einrichtungen zum kontinuierlichen Überwachen der Intensität des Laserstrahls an längs der Vorrichtung positionierten Punkten aufweist und das Steuersystem erste und zweite Detektoren (45, 41), die längs des Übertragungsbündels positioniert sind, und eine Steuereinrichtung (26) umfaßt, die in Abhängigkeit vom ersten oder zweiten Detektor betätigbar ist zwecks Unterbrechung des Durchtritts des Laserstrahls zum Gerät beim Auftreten eines anomalen Pegels oder Werts der Laserstrahlintensität am betreffenden Detektor (45), dadurch gekennzeichnet, daß das Gerät ein Katheter der Art zur Einführung in ein Blutgefäß zwecks Übertragung der Laserstrahlenergie auf einen (Gefäß-) Verschluß ist, das Steuersystem die Intensität des Laserstrahls an der distalen Spitze (13) des Katheters überwacht, das Steuersystem einen Strahlteiler (31) zum Umlenken eines Teils des zur distalen Spitze (13) geleiteten Laserstrahls auf den ersten Detektor (45) aufweist, an der distalen Spitze im Strahlengang des Laserstrahls eine fluoreszente optisch reaktive Einrichtung (37) zum Übertragen bzw. Durchlassen praktisch des gesamten Laserstrahls durch die reaktive Einrichtung angeordnet ist, die reaktive Einrichtung zum Erzeugen von Strahlungsenergie einer Wellenlänge, die beträchtlich oder deutlich verschieden ist von derjenigen des Laserstrahls, aus einem Teil des Laserstrahls dient und so angeordnet ist, daß die erzeugte Strahlungsenergie während der Übertragung des Laserstrahls über das Übertragungsbündel zum Strahlteiler (31) geleitet wird, der zweite Detektor (41) zum Abnehmen der durch den Strahlteiler umgelenkten Strahlungsenergie angeordnet ist und das Steuersystem zum Unterbrechen des Leitens oder Führens des Laserstrahls zum Katheter, wenn das Verhältnis der Übertragungsbündel-Ausgangsleistung zur Laserquellen-Ausgangsleistung außerhalb eines vorbestimmten Bereichs liegt, dient.

2. Laserkathetervorrichtung nach Anspruch 1, dadurch gekennzeichnet, daß die Steuereinrichtung (26) eine Blendeneinrichtung zum Unterbrechen des Durchtritts des Laserstrahls zwischen der (Laser-) Quelle (14) und dem Strahlteiler (31) aufweist.

3. Laserkathetervorrichtung nach Anspruch 1 oder 2, bei dem es sich um einen lasergestützten Durchleuchtungs-Gefäßplastikkatheter handelt, dadurch gekennzeichnet, daß das Übertragungsbündel einen (Faseroptik-)Lichtleiter (35) zum Übertragen des Laserstrahls vom proximalen Ende zum distalen Spitzenende des Katheters aufweist und der Strahlteiler (31) am proximalen Ende des Katheters angeordnet ist.

4. Laserkathetervorrichtung nach Anspruch 1, 2 oder 3, dadurch gekennzeichnet, daß der Strahlteiler (31) ein dichroitisches Filter ist.

5. Laserkathetervorrichtung nach Anspruch 4, dadurch gekennzeichnet, daß das fluoreszente Element (37) einen Dotierstoff enthält und so angeordnet oder ausgelegt ist, daß es bei einer Wellenlänge, die größer ist als die des einfallenden Laserstrahls, fluoresziert.

6. Laserkathetervorrichtung nach einem der vorangehenden Ansprüche, dadurch gekennzeichnet, daß das Übertragungsbündel (16) voneinander beabstandete (Faseroptik-)Lichtleitersegmente (20, 35) umfaßt und der Strahlteiler (31) zwischen diese Segmente eingefügt ist.

## Revendications

1. Dispositif médical à laser (10) comprenant une colonne de transmission (16) entre une source laser (14) et un instrument (11), le dispositif (10) ayant un système de commande muni de moyens pour contrôler continuellement l'intensité du faisceau laser en des points situés le long du dispositif, le système de commande ayant un premier détecteur et un deuxième détecteur (45, 41) placés le long de la colonne de transmission, et des moyens de commande (26) pouvant être actionnés en réponse au premier ou au deuxième détecteur pour interrompre le passage du faisceau laser vers l'instrument (11) quand survient un niveau anormal de l'intensité du faisceau laser sur le détecteur respectif (45), **caractérisé** en ce que ledit instrument (11) est un cathéter du type destiné à être inséré dans un vaisseau sanguin pour transmettre le faisceau laser sur une obstruction, le système de commande contrôle l'intensité du faisceau laser à l'extrémité distale (13) du cathéter, le système de commande comprend un diviseur de faisceau (31) pour dévier une fraction du faisceau laser dirigé sur l'extrémité distale (13) vers le premier détecteur (45), un moyen fluorescent optiquement réactif (37) disposé sur l'extrémité distale (13) sur le trajet dudit faisceau laser pour transmettre substantiellement tout le faisceau laser à travers ledit moyen réactif (37), ledit moyen réactif servant à émettre de l'énergie radiante d'une longueur d'onde nettement différente de celle du faisceau laser, à partir d'une fraction dudit faisceau laser, et étant agencé de telle sorte que ladite énergie radiante émise soit transmise le long de la colonne de transmission au diviseur de faisceau (31) pendant la transmission du faisceau laser, le deuxième détecteur (41) étant agencé pour recevoir l'énergie radiante déviée par le diviseur de faisceau, et le système de commande sert à interrompre le passage du faisceau laser vers le cathéter quand le rapport entre l'intensité en sortie de la colonne de transmission et l'intensité en sortie de la source laser tombe en dehors d'une gamme prédéterminée.

2. Dispositif cathéter à laser selon la revendication 1, dans lequel le moyen de commande comprend un moyen obturateur pour interrompre le passage du faisceau laser entre la source (14) et le diviseur de faisceau (31).

3. Dispositif cathéter à laser selon la revendication 1 ou 2, qui est un cathéter pour angioplastie transluminale, équipé d'un laser, la colonne de transmission comprenant une fibre optique (35) pour transmettre le faisceau laser depuis l'extrémité proximale du cathéter jusqu'à son extrémité distale, le diviseur de faisceau (31) étant situé à l'extrémité proximale dudit cathéter.

4. Dispositif cathéter à laser selon la revendication 1, 2 ou 3, dans lequel le diviseur de faisceau (31) est un filtre dichroïque.

5. Dispositif cathéter à laser selon la revendication 4, dans lequel l'élément fluorescent (37) contient un dopant et est agencé pour émettre par fluorescence à une longueur d'onde plus grande que celle du faisceau laser incident.

6. Dispositif cathéter à laser selon l'une quelconque des revendications précédentes dans lequel la colonne de transmission (16) comprend des segments de fibre optique espacés entre eux (20, 35) et le diviseur de faisceau (31) est interposé entre lesdits segments.

Fig. 2

Fig. 3